# EUROPEAN PATENT SPECIFICATION

(11) **EP 2 716 296 B1**
(45) Date of publication and mention of the grant of the patent: **26.04.2017**
(21) Application number: 11866924.1
(22) Date of filing: 04.11.2011
(51) Int. Cl.: A61K 36/899, A61P 25/24, A23L 33/00

(54) **NOVEL USAGE OF RICE BRAN, OR RICE HULL EXTRACT AS HISTAMINE RECEPTOR ANTAGONIST**
NEUE VERWENDUNG EINES REISKLEIE- ODER SPREUEXTRAKTES ALS HISTAMINREZEPTORANTAGONIST
NOUVELLE UTILISATION DU SON DE RIZ OU D'UN EXTRAIT DE BALLE EN TANT QU'ANTAGONISTE DE RÉCEPTEUR DE L'HISTAMINE

(30) Priority: 27.05.2011 KR 20110050472; 27.05.2011 KR 20110050474; 13.06.2011 KR 20110056624
(43) Date of publication of application: 09.04.2014
(73) Proprietor: Korea Food Research Institute, Seongnam-si, Gyeonggi-do 463-420 (KR)
(72) Inventor: CHO, Suengmok, Seongnam-si Gyeonggi-do 463-966 (KR); HAN, Daeseok, Seongnam-si Gyeonggi-do 463-410 (KR); KIM, Dong-Soo, Seoul 138-929 (KR); BAEK, Nam-In, Suwon-si Gyeonggi-do 443-767 (KR); JIN, Young-Ho, Seoul 143-768 (KR); HAN, Jin-Kyu, Seoul 135-976 (KR); SUNG, Jae-Young, Seoul 139-918 (KR); KIM, In-Ho, Yongin-si Gyeonggi-do 448-710 (KR); LEE, Chang-Ho, Yongin-si Gyeonggi-do 448-783 (KR)
(74) Representative: V.O.
(86) International application number: PCT/KR2011/008374
(87) International publication number: WO 2012/165731

(56) References cited:
- EP-A1- 0 620 007
- WO-A2-2009/145424
- CN-A- 101 538 519
- CN-A- 101 608 156
- CN-A- 101 869 237
- CN-A- 101 912 115
- JP-A- 2008 295 380
- KR-A- 20090 007 146
- KR-A- 20100 030 403
- US-A1- 2005 249 823
- KIM HYUNG-MIN ET AL: "The evaluation of the antianaphylactic effect of Oryza sativa L. subsp. hsien Ting in rats", PHARMACOLOGICAL RESEARCH, ACADEMIC PRESS, LONDON, GB, vol. 40, no. 1, 1 July 1999 (1999-07-01), pages 31-36, XP009101721, ISSN: 1043-6618, DOI: 10.1006/PHRS.1998.0386
- SEUNG-HO LEE ET AL: "Inhibitory Effect of DA-9201, an Extract of Oryza sativa L., on Airway Inflammation and Bronchial Hyperresponsiveness in Mouse Asthma Model", BIOLOGICAL AND PHARMACEUTICAL BULLETIN, vol. 29, no. 6, 1 January 2006 (2006-01-01), pages 1148-1153, XP055135717,
- CHOI HYUN-IM ET AL: "Anticancer (in vitro) and Antiallergy Effects of Rice Bran Extracts", HAN'GUG SIGPUM YEONG'YANGGWAHAG HOEJI - JOURNAL OF THE KOREAN SOCIETY OF FOOD SCIENCE AND NUTRITION, HAN'GUG YEONG'YANG SIGRYANG HAGHOE, PUSAN, KR, vol. 35, no. 10, 29 December 2006 (2006-12-29), pages 1297-1303, XP053026450, ISSN: 1226-3311
- CHOI SUN-PHIL ET AL: "Inhibitory Activity of Pigmented Rice Bran Extract to the Allergic Inflammation in Basophilic Cell Line and Peritoneal Mast Cells", HAN'GUG EUNG'YONG SAENGMYEONG HWA HAGHOEJI - JOURNAL OF THE KOREAN SOCIETY FOR APPLIED BIOLOGICAL CHEMISTRY, KOREAN SOCIETY FOR APPLIED BIOLOGICAL CHEMISTRY, SEOUL, KOREA, vol. 48, no. 4, 31 December 2005 (2005-12-31), pages 315-321, XP053021132, ISSN: 1738-2203
- KIM, H-M. ET AL.: 'The evaluation of the antianaphylactic effect of Oryza Sativa L. subsp. Hsien ting in Rats' PHARMACOLOGICAL RESEARCH vol. 40, no. 1, 1999, pages 31 - 36, XP009101721
- LEE, S-H. ET AL.: 'Inhibitory effect of DA-9201, an extract of Oryza sativa L., on airway inflammation and bronchial hyperresponsiveness in mouse asthma model' BIOLOGICAL AND PHARMACEUTICAL BULLETIN vol. 29, no. 6, 2006, pages 1148 - 1153, XP055135717
- HYUN IM CHOI ET AL.: 'Anticancer (in vitro) and Antiallergy Effects of Rice Bran Extracts' JOURNAL OF THE KOREAN SOCIETY OF FOOD SCIENCE AND NUTRITION vol. 35, no. 10, 2006, pages 1297 - 1303, XP053026450
- SUN PHIL CHOI ET AL.: 'Inhibitory Activity of Pigmented Rice Bran Extract to the Allergic Inflammation in Basophilic Cell Line and Peritoneal Mast Cells' JOURNAL OF THE KOREAN SOCIETY FOR APPLIED BIOLOGICAL CHEMISTRY vol. 48, no. 4, 2005, pages 315 - 321, XP053021132
- PASSANI, M. B. ET AL.: 'Histamine receptors in the CNS as targets for therapeutic intervention' TRENDS IN PHARMACOLOGICAL SCIENCES vol. 32, no. 4, April 2011, pages 242 - 249, XP028196562

## Description

### TECHNICAL FIELD

The present disclosure relates to the use of rice bran or rice hull extract as a histamine receptor antagonist. In particular, it relates to a composition containing rice bran or rice hull extract as an active ingredient for preventing or treating sleep disorder.

### BACKGROUND

The average person spends 1/3 of his/her lifetime sleeping. Sleep is the most basic and essential physiological process, important in health maintenance and mental stability. Chronic sleep deficiency and disorder have negative effects on physical and mental health, including cardiovascular diseases and hypertension, memory and learning, metabolic regulation and body weight, immunity and resistance to cancer, diabetes, negligent accident, mood, etc. Currently, an estimated 30% of the world population are affected by insomnia, and 10% have chronic insomnia. The sleep disorder is becoming an important issue.

According to the survey by the National Sleep Foundation in 2008, about 50% of American adults are affected by insomnia at least once a week, and 50% of American people are not satisfied with their sleep. Recently, the prevalence of insomnia and sleep disorder in Korea is increasing fast close to the level of developed countries, with the patients treated for sleep disorder increasing 4.5-fold in 8 years from 51,000 in 2001 to 228,000 in 2008 (National Health Insurance Corporation, 2009).

In Korea, sleep disorder is not yet recognized as a disease requiring medical treatment in general. But, it is reported that about 15% of adults require medication because of insomnia caused by anxiety. The causes of insomnia include stress, tension, terror, etc., and benzodiazepine drugs and serotonin acting drugs are used to treat insomnia. However, long-term use of these drugs leads to severe side effects including cognitive impairment as well as drug resistance and dependency. In the US, antihistamines and natural herbs with less side effects and dependency are used as non-prescription sleep aids. Especially, antihistamine, which is a histamine receptor antagonist, is the ingredient of cold medicine and is the only over-the-counter sleeping drug that can be purchased without prescription.

Histamine [2-(4-imidazolyl)ethylamine] is one of the neurotransmitters widely distributed throughout the body, e.g. in the gastrointestinal tract [Burks 1994 in Johnson L.R. ed., Physiology of the Gastrointestinal Tract, Raven Press, NY, pp. 211-242]. Histamine regulates various pathophysiological events including gastric acid secretion, bowel movement [see Leurs et al., Br. J. Pharmacol. 1991, 102, pp. 179-185], response of the vasomotor system, inflammatory response and allergic reaction [see Raithel et al., Int. Arch. Allergy Immunol. 1995, 108, 127-133] [see Panula et al., Proc. Natl. Acad. Sci. USA 1984, 81, 2572-2576; Inagaki et al., J. Comp. Neurol. 1988, 273, 283-300]. The action of histamine in the central and peripheral nervous systems is mediated by the four currently known histamine receptors, i.e. H₁, H₂, H₃ and H₄ receptors. The histamine receptors H₁, H₂, H₃ and H₄ are known to be involved in allergic and immune responses such as allergic rhinitis, inflammatory bowel disease, asthma, bronchitis and nausea, in gastric and duodenal ulcer or gastroesophageal reflux disease related with gastric acid secretion, and in sedation and sleep inducement in the brain, by acting alone or in combination.

Rice is one of the world's three major grains and is a valuable staple food for the half of the world's population. Especially, it is more important than any other grains in Asia. Unpolished rice with the rice hull removed is called brown rice, and the rice polished by removing the rice bran and rice germ is called white rice. Although the rice bran is the byproduct of the rice milling process, it contains various nutrients.

The nutrients contained in the rice bran are about 95% of those of rice, including high-quality proteins, dietary fiber, vitamins and minerals. The rice bran is known to have anticancer, antioxidant, anti-inflammatory, anti-arteriosclerotic, cholesterol-reducing, growth-promoting, digestion-promoting and immune-enhancing activities. However, the relationship of rice, rice bran or rice hull with histamine receptor antagonists or with diseases related with sleep has not been known yet.

KR 2010 0030403 is directed to the medical use of a composition comprising germinated brown rice extract, ginseng extract and Longanae Arillus extract. No mention is made in this document of rice bran or rice hull extracts.

### SUMMARY

The present disclosure is directed to providing a histamine receptor antagonist derived from a natural product, which can replace the existing drugs used for prevention or treatment of sleep disorder

In one general aspect, the present disclosure provides a food composition for preventing or improving sleep disorder, which contains rice bran or rice hull extract as an active ingredient.

### BRIEF DESCRIPTION OF THE DRAWINGS

The above and other objects, features and advantages of the present disclosure will become apparent from the following description of certain exemplary embodiments given in conjunction with the accompanying drawings, in which:

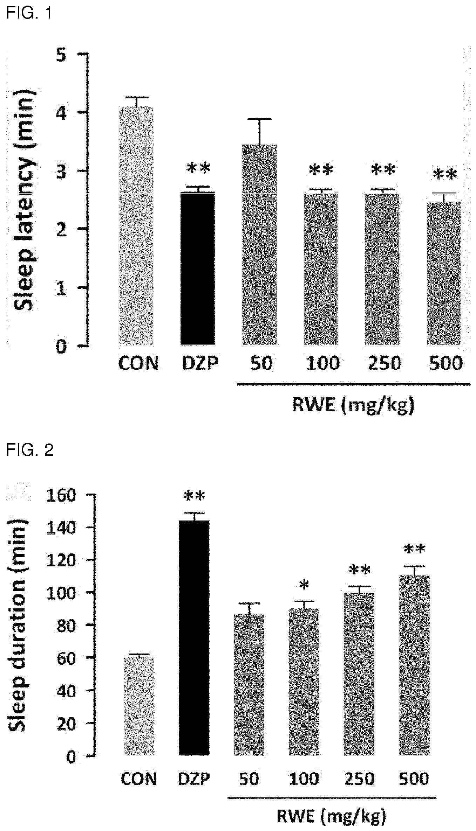

FIG. 1 shows an effect of rice water extract (RWE) on sleep latency in mouse to which a hypnotic dosage (45 mg/kg, i.p.) of pentobarbital was administered, and FIG. 2 shows an effect of RWE on sleep duration in mouse to which a hypnotic dosage (45 mg/kg, i.p.) of pentobarbital was administered [Control substance (0.5% CMC-saline 10 mL/kg), DZP (2 mg/kg) or RWE (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

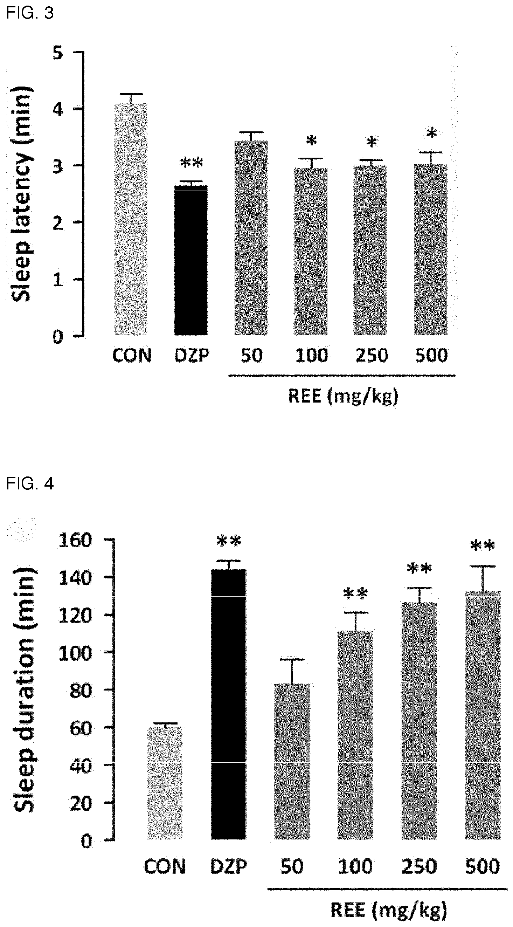

FIG. 3 shows an effect of rice ethanol extract (REE) on sleep latency in mouse to which a hypnotic dosage (45 mg/kg, i.p.) of pentobarbital was administered, and FIG. 4 shows an effect of REE on sleep duration in mouse to which a hypnotic dosage (45 mg/kg, i.p.) of pentobarbital was administered [Control substance (0.5% CMC-saline 10 mL/kg), DZP (2 mg/kg) or REE (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

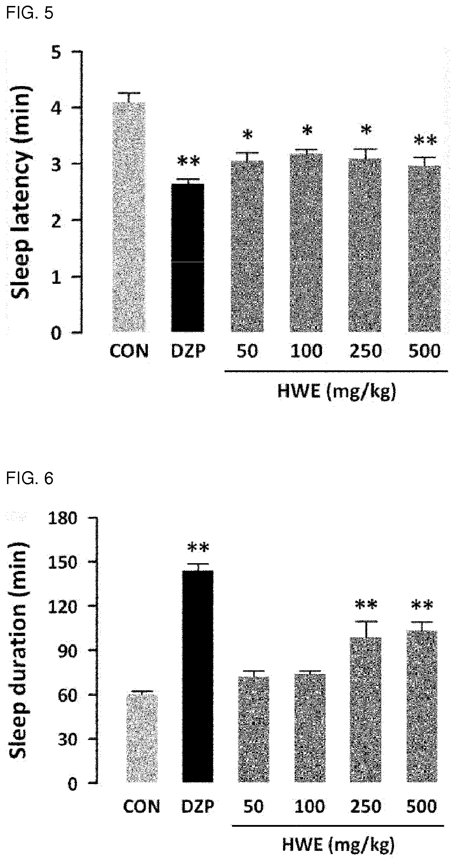

FIG. 5 shows an effect of rice hull water extract (HWE) on sleep latency in mouse to which a hypnotic dosage (45 mg/kg, i.p.) of pentobarbital was administered, and FIG. 6 shows an effect of HWE on sleep duration in mouse to which a hypnotic dosage (45 mg/kg, i.p.) of pentobarbital was administered [Control substance (0.5% CMC-saline 10 mL/kg), DZP (2 mg/kg) or HWE (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

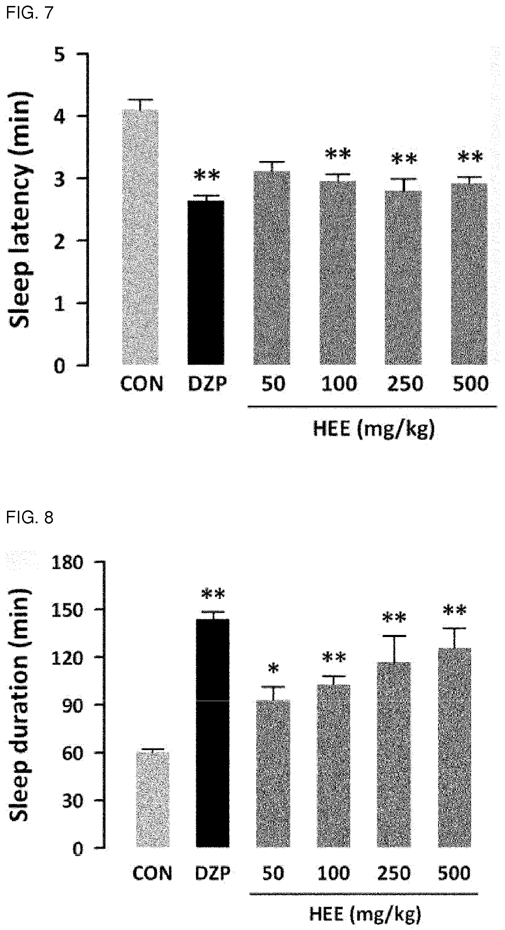

FIG. 7 shows an effect of rice hull ethanol extract (HEE) on sleep latency in mouse to which a hypnotic dosage (45 mg/kg, i.p.) of pentobarbital was administered, and FIG. 8 shows an effect of HEE on sleep duration in mouse to which a hypnotic dosage (45 mg/kg, i.p.) of pentobarbital was administered [Control substance (0.5% CMC-saline 10 mL/kg), DZP (2 mg/kg) or HEE (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

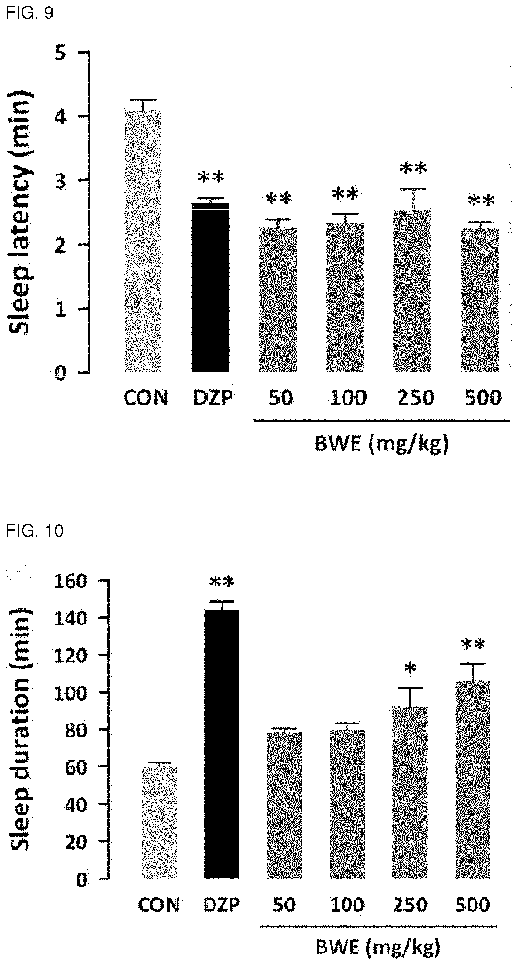

FIG. 9 shows an effect of rice bran water extract (BWE) on sleep latency in mouse to which a hypnotic dosage (45 mg/kg, i.p.) of pentobarbital was administered, and FIG. 10 shows an effect of BWE on sleep duration in mouse to which a hypnotic dosage (45 mg/kg, i.p.) of pentobarbital was administered [Control substance (0.5% CMC-saline 10 mL/kg), DZP (2 mg/kg) or BWE (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

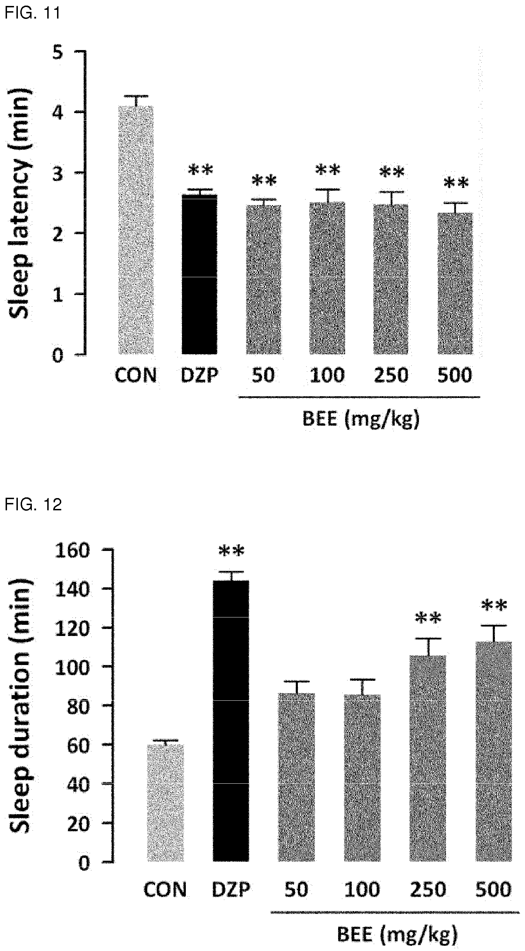

FIG. 11 shows an effect of rice bran ethanol extract (BEE) on sleep latency in mouse to which a hypnotic dosage (45 mg/kg, i.p.) of pentobarbital was administered, and FIG. 12 shows an effect of BEE on sleep duration in mouse to which a hypnotic dosage (45 mg/kg, i.p.) of pentobarbital was administered [Control substance (0.5% CMC-saline 10 mL/kg), DZP (2 mg/kg) or BEE (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

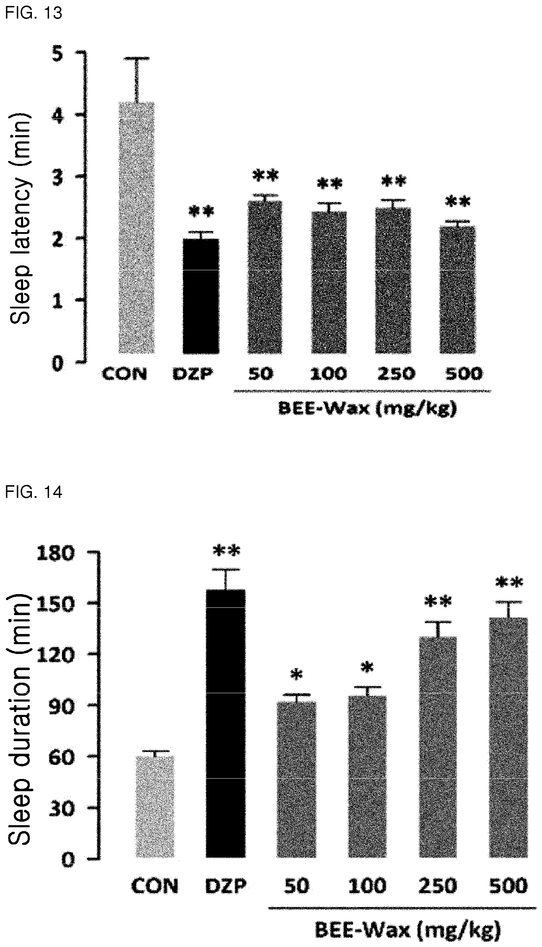

FIG. 13 shows an effect of a wax fraction of rice bran extract (BEE-Wax) on sleep latency in mouse to which a hypnotic dosage (45 mg/kg, i.p.) of pentobarbital was administered, and FIG. 14 shows an effect of BEE-Wax on sleep duration in mouse to which a hypnotic dosage (45 mg/kg, i.p.) of pentobarbital was administered [Control substance (0.5% CMC-saline 10 mL/kg), DZP (2 mg/kg) or BEE-Wax (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

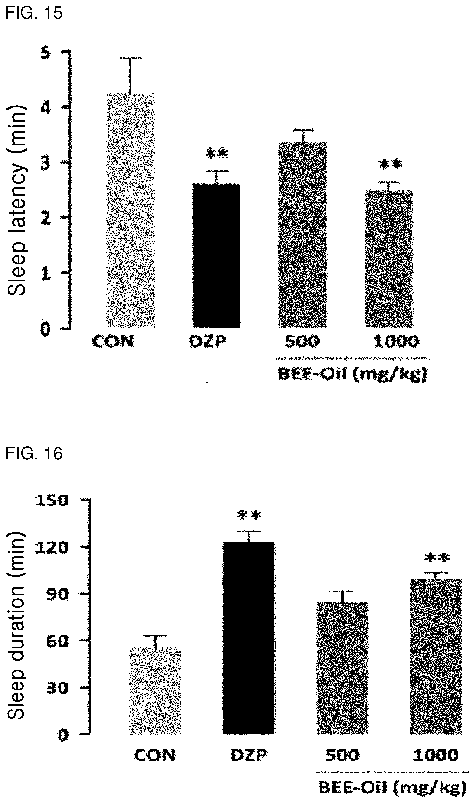

FIG. 15 shows an effect of an oil fraction of rice bran extract (BEE-Oil) on sleep latency in mouse to which a hypnotic dosage (45 mg/kg, i.p.) of pentobarbital was administered, and FIG. 16 shows an effect of BEE-Oil on sleep duration in mouse to which a hypnotic dosage (45 mg/kg, i.p.) of pentobarbital was administered [Control substance (0.5% CMC-saline 10 mL/kg), DZP (2 mg/kg) or BEE-Oil (50, 100, 250, 500 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control and DZP stands for diazepam.];

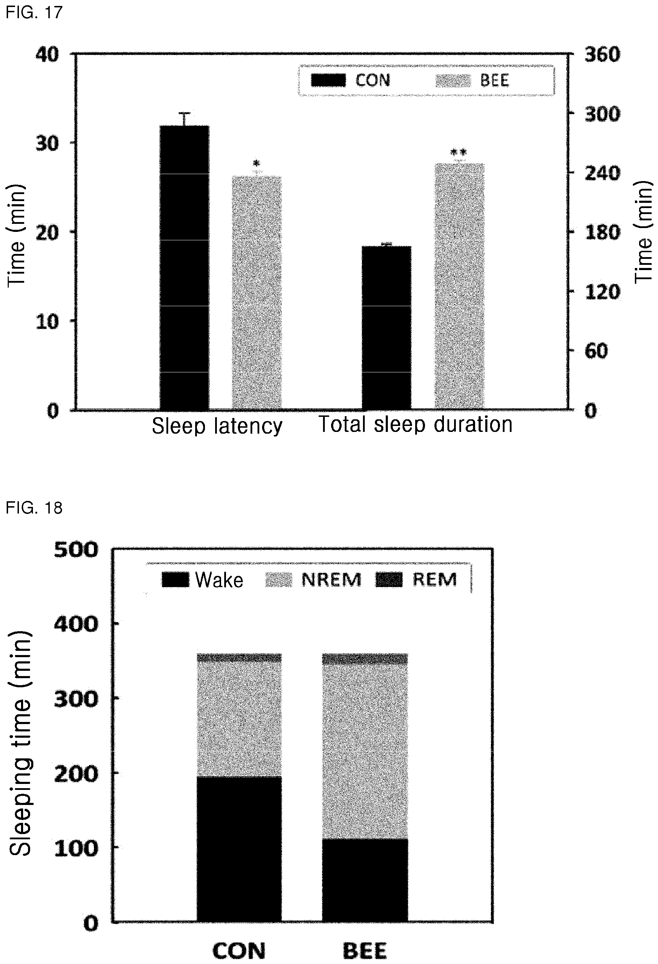

FIG. 17 shows an effect of BEE (500 mg/kg) on sleep latency and total sleep duration in SD rat [Each graph shows mean ± SEM (n = 8). * and ** indicate significant difference (Dunnett's test) as compared to the control at p < 0.05 and p < 0.01, respectively. CON stands for control.];
FIG. 18 shows an effect of BEE (500 mg/kg) on sleep architecture in SD rat [CON stands for control.];

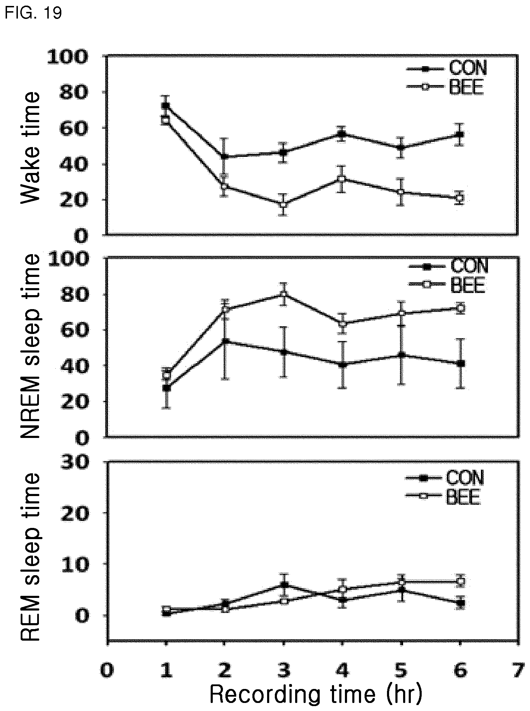

FIG. 19 shows change in wake, non-REM sleep and REM sleep with time [CON stands for control.];

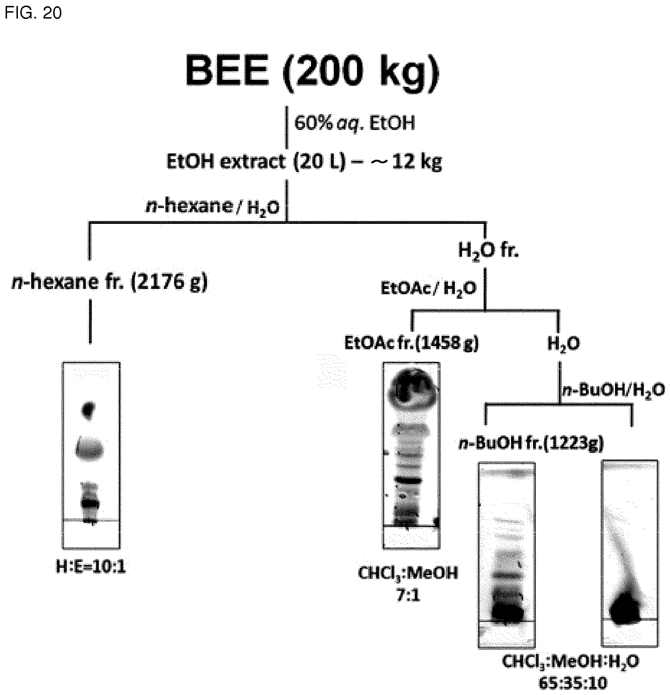

FIG. 20 shows a procedure of fractionating BEE [EtOAc stands for ethyl acetate, n-BuOH for n-butanol, and H₂O for distilled water.];

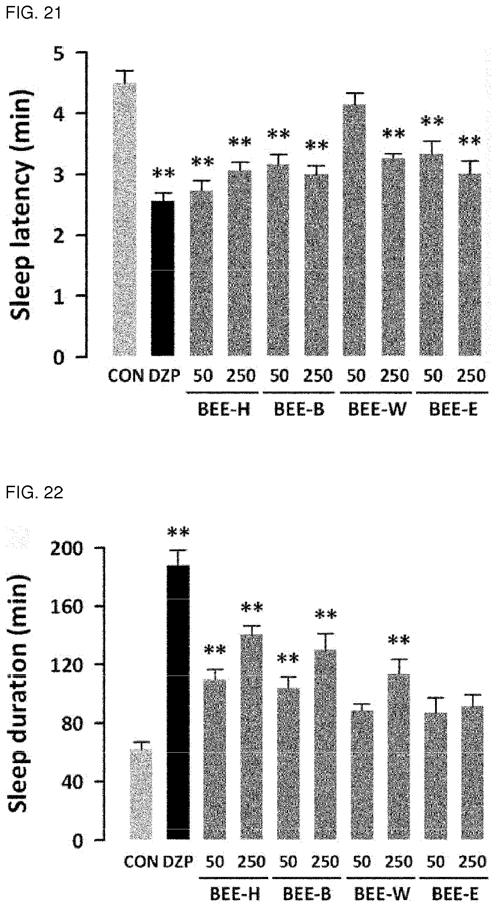

FIG. 21 shows an effect of fractions of BEE on sleep latency in mouse to which a hypnotic dosage (45 mg/kg, i.p.) of pentobarbital was administered, and FIG. 22 shows an effect of fractions of BEE on sleep duration in mouse to which a hypnotic dosage (45 mg/kg, i.p.) of pentobarbital was administered [Control substance (0.5% CMC-saline 10 mL/kg), DZP (2 mg/kg) or BEE-H, BEE-B, BEE-W or BEE-E (50, 250 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. CON stands for control, DZP for diazepam, H for hexane extract, B for butanol extract, W for water extract, and E for ethyl acetate extract.];

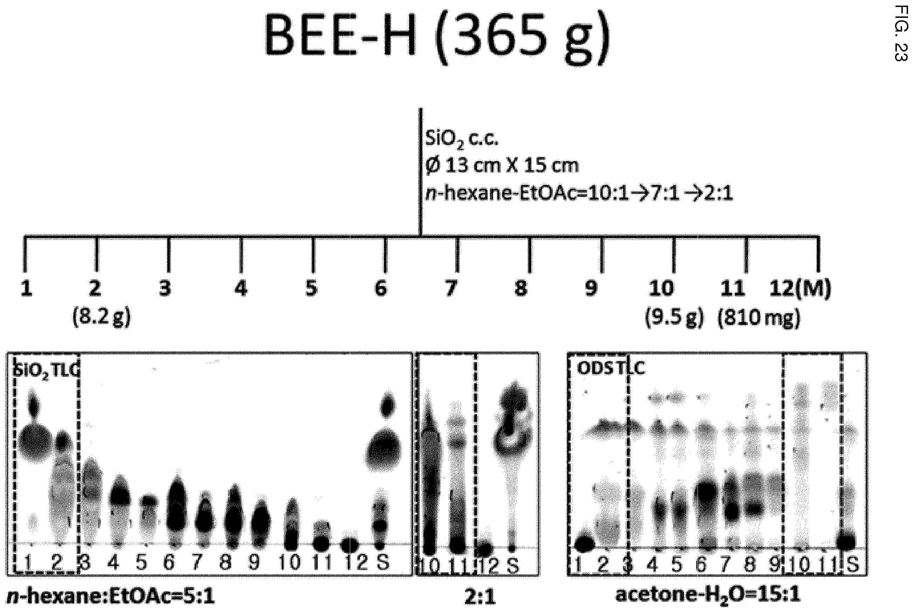

FIG. 23 shows a procedure of separating sub-fractions of a n-hexane extract of BEE [EtOAc stands for ethyl acetate and H₂O stands for distilled water.];

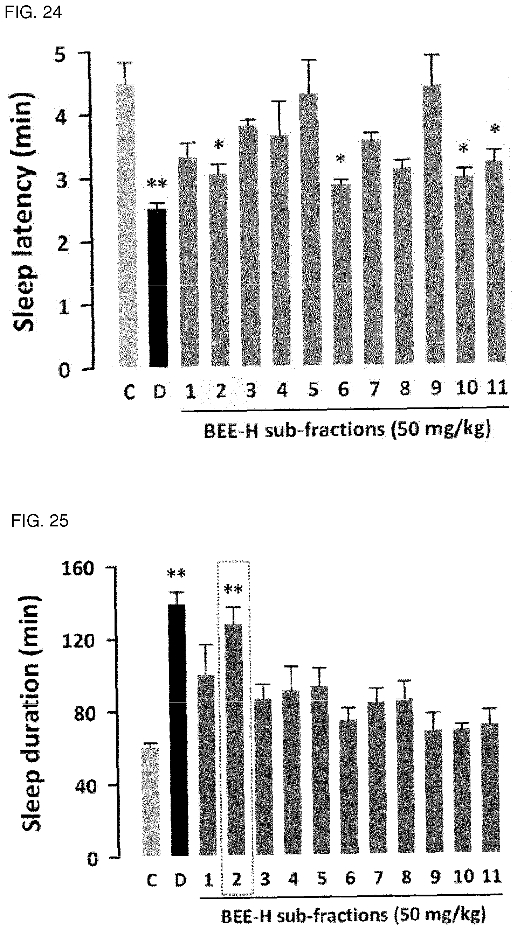

FIG. 24 shows an effect of sub-fractions of BEE-H on sleep latency in mouse to which a hypnotic dosage (45 mg/kg, i.p.) of pentobarbital was administered, and FIG. 25 shows an effect of sub-fractions of BEE-H on sleep duration in mouse to which a hypnotic dosage (45 mg/kg, i.p.) of pentobarbital was administered [Control substance (0.5% CMC-saline 10 mL/kg), DZP (2 mg/kg) or each BEE-H sub-fraction (50 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. C stands for control and D stands for diazepam.];

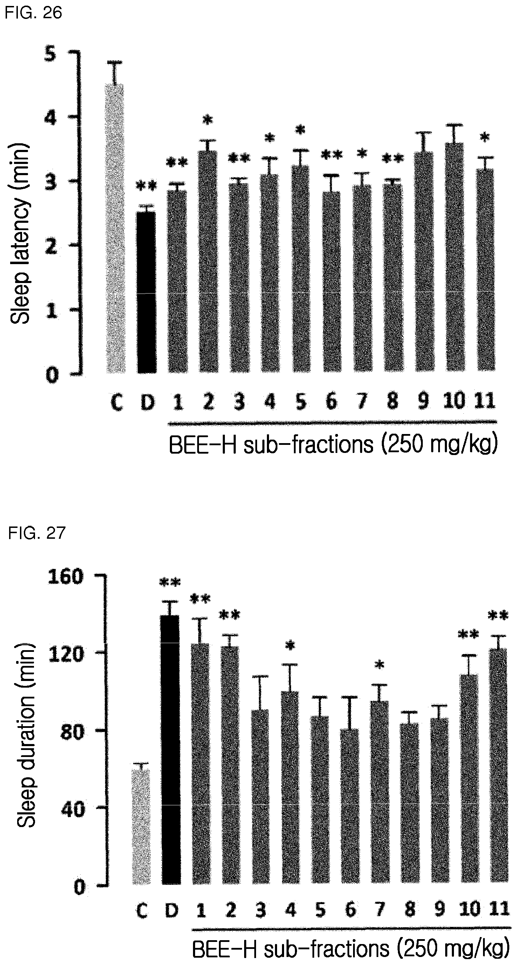

FIG. 26 shows an effect of sub-fractions of BEE-H on sleep latency in mouse to which a hypnotic dosage (45 mg/kg, i.p.) of pentobarbital was administered, and FIG. 27 shows an effect of sub-fractions of BEE-H on sleep duration in mouse to which a hypnotic dosage (45 mg/kg, i.p.) of pentobarbital was administered [Control substance (0.5% CMC-saline 10 mL/kg), DZP (2 mg/kg) or each BEE-H sub-fraction (250 mg/kg) was orally administered (p.o.) and pentobarbital was administered 45 minutes later. Each graph shows mean ± SEM (n = 10). * and ** indicate significant difference (Dunnett's test) as compared to the control at p < 0.05 and p < 0.01, respectively. C stands for control and D stands for diazepam.];

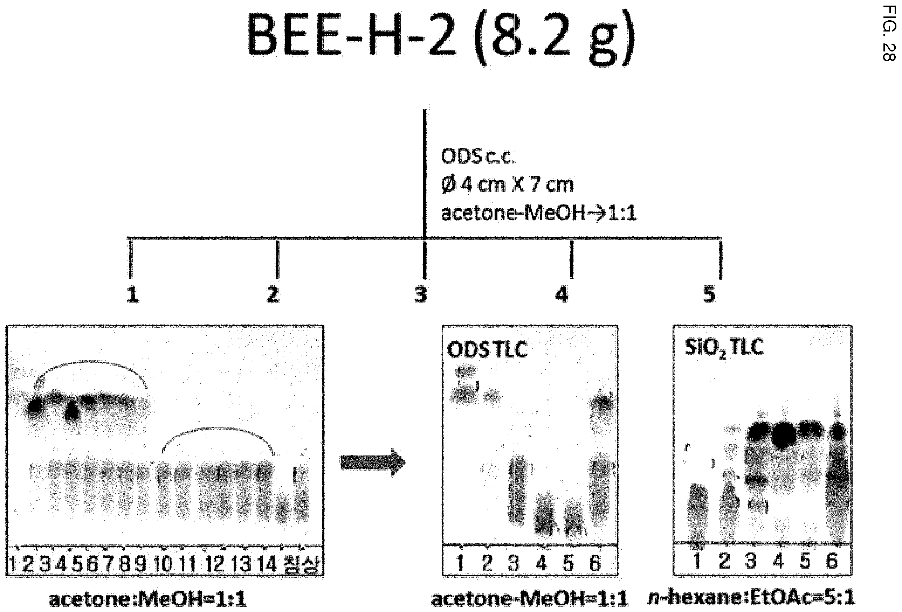

FIG. 28 shows a procedure of separating sub-fractions of a n-hexane extract of BEE [MeOH stands for methanol and EtOAc stands for ethyl acetate.];

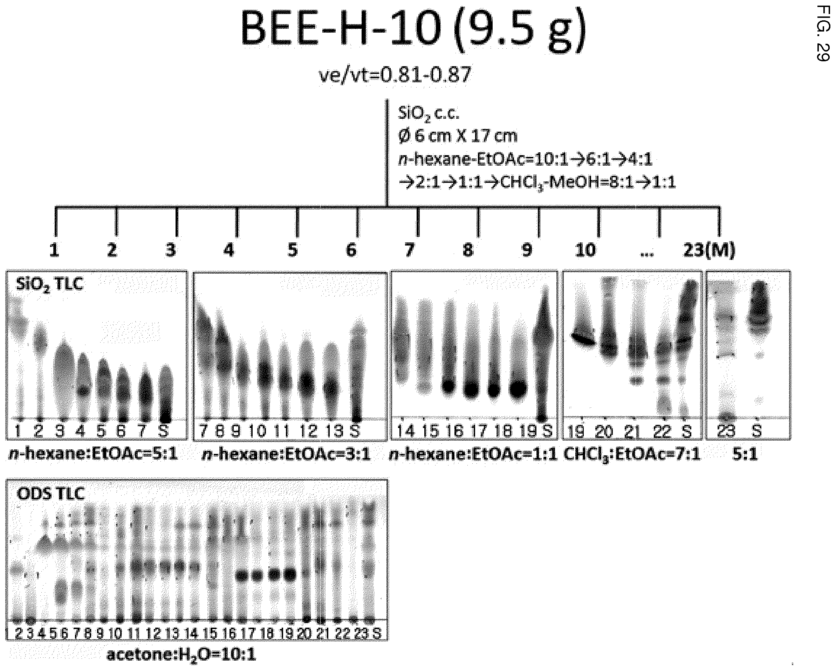

FIG. 29 shows a procedure of separating sub-fractions of a n-hexane extract of BEE [EtOAc stands for ethyl acetate, CHCl₃ for chloroform, MeOH for methanol, and H₂O for distilled water.];

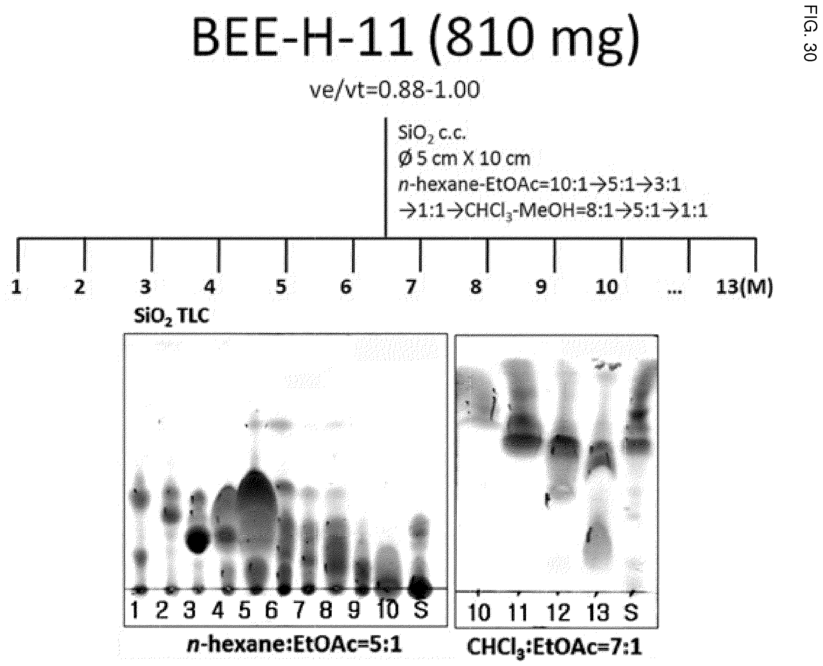

FIG. 30 shows a procedure of separating sub-fractions of a n-hexane extract of BEE [EtOAc stands for ethyl acetate, CHCl₃ for chloroform, and MeOH for methanol.];

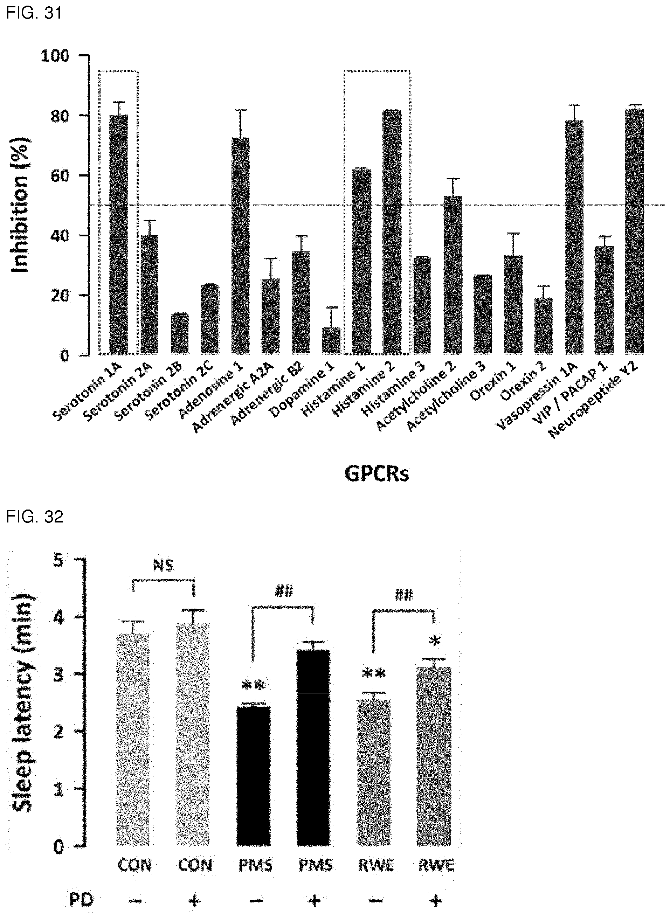

FIG. 31 shows an effect of the BEE-H-2 fraction on the activity of G protein-coupled receptors (GPCRs);

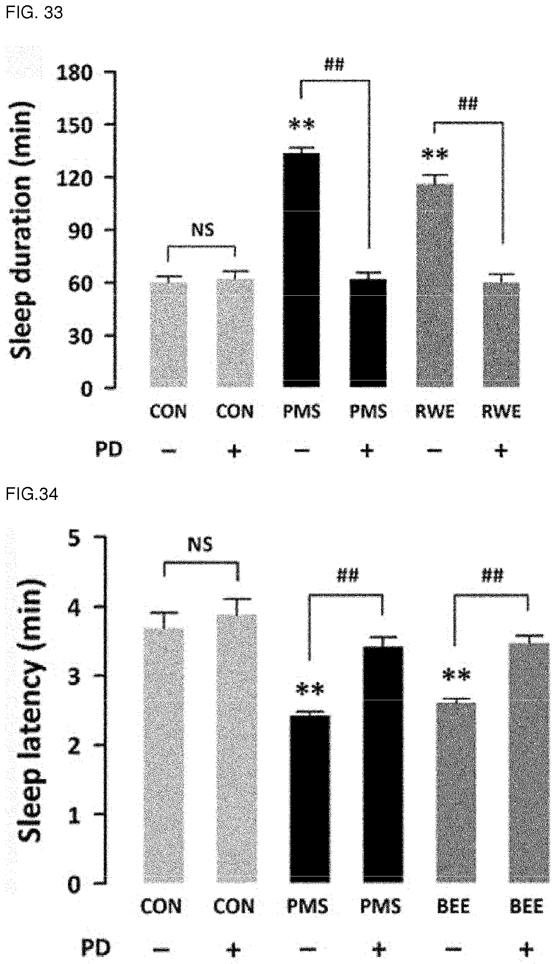

FIG. 32 shows an effect of the RWE prepared in Preparation Example 1 and a histamine receptor antagonist (PMS) on sleep latency in mouse to which a histamine receptor agonist (PD) was administered, and FIG. 33 shows an effect of the RWE prepared in Preparation Example 1 and PMS on sleep duration in mouse to which PD was administered [Control substance (CON, 0.5% CMC-saline 10 mL/kg), RWE (500 mg/kg) or PMS (70 mg/kg) was orally administered and a hypnotic dosage (45 mg/kg) of pentobarbital was administered 45 minutes later. * and ** indicate significant difference (Dunnett's test) as compared to the control at p < 0.05 and p < 0.01, respectively.];

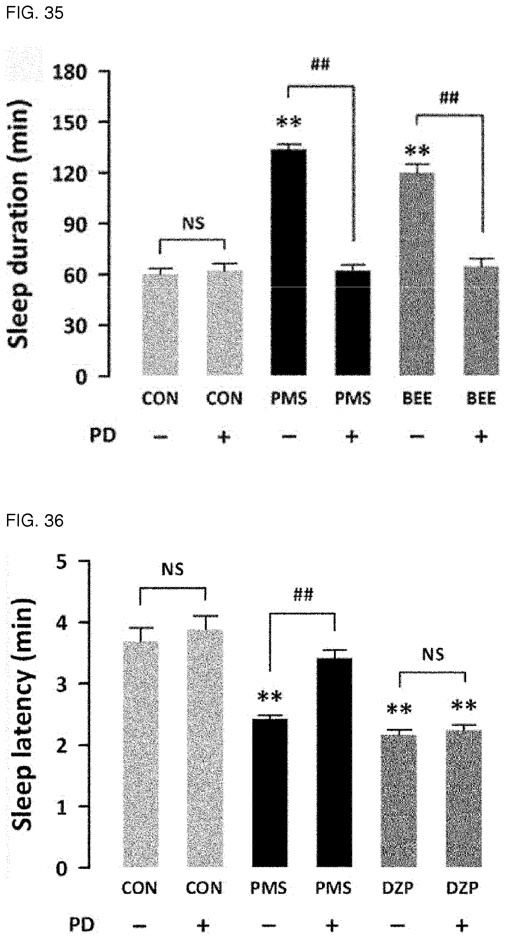

FIG. 34 shows an effect of the BEE prepared in Preparation Example 3 and a histamine receptor antagonist (PMS) on sleep latency in mouse to which a histamine receptor agonist (PD) was administered, and FIG. 35 shows an effect of the BEE prepared in Preparation Example 3 and PMS on sleep duration in mouse to which PD was administered [Control substance (CON, 0.5% CMC-saline 10 mL/kg), BEE (500 mg/kg) or PMS (70 mg/kg) was orally administered and a hypnotic dosage (45 mg/kg) of pentobarbital was administered 45 minutes later. * and ** indicate significant difference (Dunnett's test) as compared to the control at p < 0.05 and p < 0.01, respectively.];

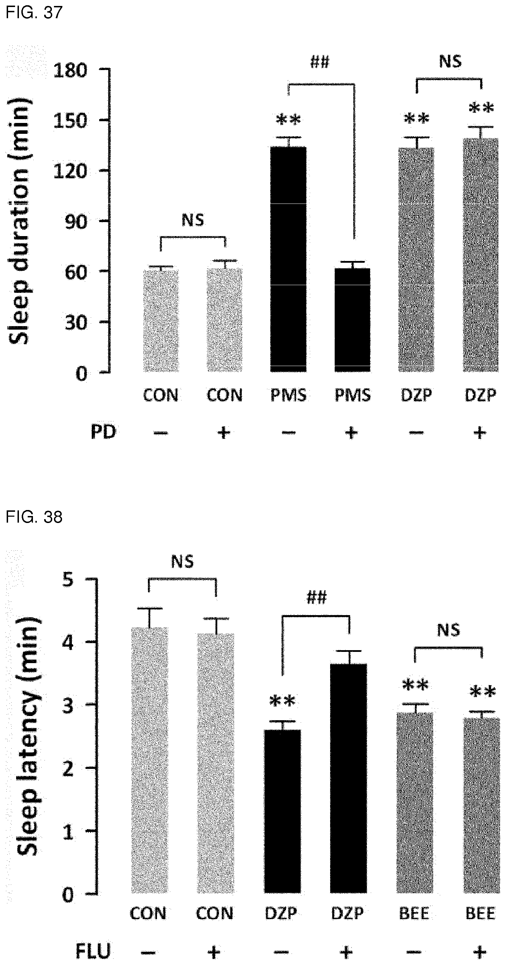

FIG. 36 shows an effect of diazepam (DZP) and PMS on sleep latency in mouse to which a histamine receptor agonist (PD) was administered, and FIG. 37 shows an effect of DZP and PMS on sleep duration in mouse to which PD was administered [Control substance (CON, 0.5% CMC-saline 10 mL/kg), DZP (2 mg/kg) or PMS (70 mg/kg) was orally administered and a hypnotic dosage (45 mg/kg) of pentobarbital was administered 45 minutes later. * and ** indicate significant difference (Dunnett's test) as compared to the control at p < 0.05 and p < 0.01, respectively.]; and

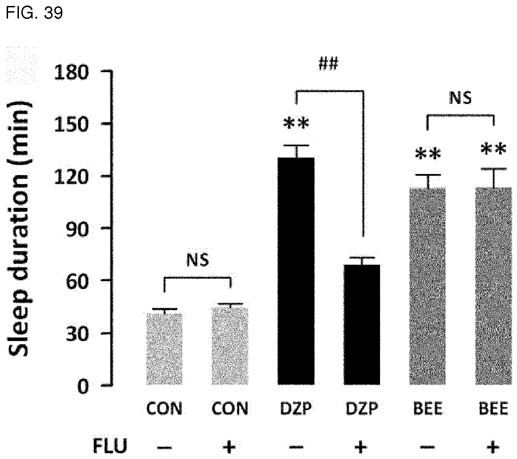

FIG. 38 shows an effect of diazepam (DZP) and BEE on sleep latency in mouse to which the GABAA-benzodiazepine antagonist flumazenil (FLU) was administered, and FIG. 39 shows an effect of DZP and BEE on sleep duration in mouse to which FLU was administered [Control substance (CON, 0.5% CMC-saline 10 mL/kg), DZP (2 mg/kg) or BEE (500 mg/kg) was orally administered and a hypnotic dosage (45 mg/kg) of pentobarbital was administered 45 minutes later. FLU (8 mg/kg) was abdominally injected 10 minutes before the oral administration of DZP or BEE. * and ** indicate significant difference (Dunnett's test) as compared to the control at p < 0.05 and p < 0.01, respectively.].

### DETAILED DESCRIPTION OF EMBODIMENTS

As used herein the term "agonist" refers to a substance that interacts with a histamine receptor, i.e. H₁, H₂, H₃ or H₄ receptor, to activate the histamine receptor and triggers a physiological or pharmacological response of the receptor, and the term "antagonist" refers to a substance that binds to the receptor at the same site competitively with the agonist without activating the intracellular response initiated by the activated form of the receptor and thus inhibits the intracellular response triggered by the agonist, unless specified otherwise.

The inventors of the present disclosure have made efforts to prepare a natural composition of a histamine receptor antagonist effective for prevention or treatment of sleep disorder. As a result, they have elucidated that rice bran acts as histamine receptor antagonist and is effective for the prevention or treatment of sleep disorder.

The present disclosure provides a novel use of rice bran or rice hull extract as a histamine receptor antagonist, a pharmaceutical composition for preventing or treating sleep disorder comprising rice, rice bran or rice hull extract as an active ingredient. For preventing or treating sleep disorder, a therapeutically effective amount of rice bran extract or rice bran powder is to be administered to a subject.

As used herein, the prevention or treatment of sleep disorder may mean the reduction of sleep latency, increase of sleep duration or increase of non-REM sleep.

As demonstrated through the following examples, rice bran or rice hull extract exhibits comparable or better effect of reducing sleep latency, increasing sleep duration and increasing non-REM sleep duration when compared with diazepam which is currently used to treat insomnia, anxiety or depression. Also, the effect of rice bran or rice hull extract on sleeping is inhibited by the same mechanism as that by which the effect of the histamine receptor agonist 2-pyridylethylamine dihydrochloride (PD) is completely inhibited by pyrilamine maleate salt (PMS) known as a histamine receptor antagonist, suggesting that rice bran or rice hull extract acts as a natural antihistamine. Accordingly, rice bran or rice hull extract can be used as an active ingredient of a composition for preventing or treating sleep disorder Unlike the existing sleeping drugs, rice bran or rice hull extract is unharmful foodstuff with no side effect. With superior effect of inducing sleep and increasing sleep duration, it may be usefully used for the prevention or treatment of sleep disorder. Although experiments about anxiety or depression were not carried out, it is well known to those skilled in the art that a substance used for improving, preventing or treating sleep disorder, such as diazepam, be used to improve, prevent or treat anxiety or depression by changing its administration dose.

In an exemplary embodiment of the present disclosure, the rice bran or rice hull extract may be extracted from rice bran using water, an organic solvent or a mixture thereof as an extraction solvent. The organic solvent or a mixing proportion of water with the organic solvent is not particularly limited.

For example, the organic solvent may be one or more solvent selected from a group consisting of lower alcohol, hexane, acetone, ethyl acetate, chloroform and diethyl ether. The lower alcohol may be C1-C6 alcohol. For example, the lower alcohol may be methanol, ethanol, propanol, butanol, n-propanol, isopropanol, n-butanol, 1-pentanol, 2-butoxyethanol, ethylene glycol, etc. Besides, the organic solvent may be a polar solvent such as acetic acid, dimethylformamide (DMF), dimethyl sulfoxide (DMSO), etc. or a nonpolar solvent such as acetonitrile, ethyl acetate, methyl acetate, fluoroalkane, pentane, 2,2,4-trimethylpentane, decane, cyclohexane, cyclopentane, diisobutylene, 1-pentene, 1-chlorobutane, 1-chloropentane, o-xylene, diisopropyl ether, 2-chloropropane, toluene, 1-chloropropane, chlorobenzene, benzene, diethyl ether, diethyl sulfide, chloroform, dichloromethane, 1,2-dichloroethane, aniline, diethylamine, ether, carbon tetrachloride, tetrahydrofuran (THF), etc.

As demonstrated through the following examples, when rice bran or rice hull is extracted with an organic solvent, the rice bran or rice hull extract is separated into an upper layer and a lower layer. The upper layer is an oil fraction of the rice bran extract, and the lower layer is the solvent extract. When the lower layer is further separated via centrifugation or through filter paper, it is separated into liquid phase and solid residue. The residue is the wax fraction of the rice bran or rice hull extract. Accordingly, the rice bran or rice hull extract of the present disclosure is understood as including the oil fraction of the rice bran or rice hull extract, the liquid fraction of the rice bran extract and the wax fraction of the rice bran extract. In an exemplary embodiment, the rice bran or rice hull extract may comprise one or more selected from a group consisting of the oil fraction of the rice bran or rice hull extract, the liquid fraction of the rice bran or rice hull extract and the wax fraction of the rice bran or rice hull extract.

In an exemplary embodiment, the rice bran or rice hull extract may be a lower alcohol extract of rice bran, more specifically an ethanol extract of rice bran or rice hull.

In an exemplary embodiment, the rice bran or rice hull extract may be a hexane extract of rice bran or rice hull. The hexane extract of rice bran or rice hull is commonly used as 'rice bran oil'. Accordingly, in an exemplary embodiment of the present disclosure, the rice bran extract may be rice bran oil. As used herein, the term 'extract' includes fractions of the extract obtained by further fractionation. That is to say, in addition to the extract obtained using an extraction solvent, the rice bran or rice hull extract also includes further purified fractions. Also, fractions obtained by passing the extract or fraction through an ultrafiltration membrane with a predetermined molecular weight cut-off value or purifying it by chromatography (based on size, charge, hydrophobicity or affinity) or other various purification methods are included in the rice bran or rice hull extract of the present disclosure.

In an exemplary embodiment, the rice bran or rice hull extract may be fraction obtained by refractionating an organic solvent extract of rice bran or rice hull with a second organic solvent. As used herein, the organic solvent extract of rice bran or rice hull includes, in a broad sense, the oil fraction of rice bran or rice hull extract, the liquid fraction of rice bran or rice hull extract and the wax fraction of rice bran or rice hull extract described above, and, in a narrow sense, refers to the liquid fraction of rice bran or rice hull extract among them. Accordingly, in an exemplary embodiment, the fraction obtained by refractionating an organic solvent extract of rice bran or rice hull with a second organic solvent may mean a fraction obtained by refractionating a liquid fraction of rice bran extract with a second organic solvent. In another exemplary embodiment, the rice bran or rice hull extract may be a fraction obtained by refractionating a lower alcohol extract of rice bran or rice hull with a second organic solvent. In another exemplary embodiment, the rice bran or rice hull extract may be a fraction obtained by refractionating an ethanol extract of rice bran or rice hull with hexane. As demonstrated through following examples, the fractions of rice bran or rice hull extract containing the oil-soluble components of rice bran or rice hull in large quantities exhibit very superior effect of inducing sleep and increasing sleep duration.

As used herein, the term 'extract' in relation to rice bran or rice hull includes not only a crude extract obtained by treating rice bran with an extraction solvent but also a processed product of the rice bran or rice hull extract. For example, the rice bran or rice hull extract may be prepared into powder form through further processes such as distillation under reduced pressure, lyophilization, spray drying, or the like.

Also, in a broad sense, the rice bran or rice hull extract of the present disclosure includes a product of rice bran or rice hull processed to be administrable to an animal, e.g. rice bran or rice hull powder. Although experiments were carried out only with the rice bran or rice hull extract, those skilled in the art will understand that the desired effect can be achieved also with the processed product of rice bran or rice hull.

As used herein, the term 'therapeutically effective amount' refers to an amount of the rice bran or rice hull extract sufficient to achieve the desired effect or activity. In an exemplary embodiment, the rice bran or rice hull extract is included in the composition of the present disclosure in an amount of, for example, 0.001 mg/kg or more, specifically 0.1 mg/kg or more, more specifically 10 mg/kg or more, more specifically 100 mg/kg or more, more specifically 250 mg/kg or more, most specifically 0.1 g/kg or more. Since the rice bran or rice hull extract is a natural product with no side effect even when administered in excessive quantities to the human body, the upper limit of the amount of the rice bran or rice hull extract included in the composition of the present disclosure may be adequately determined by those skilled in the art.

A pharmaceutical composition of the present disclosure may be prepared by using, in addition to the active ingredient, a pharmaceutically and physiologically acceptable adjuvant. The adjuvant may include an excipient, a disintegrant, a sweetener, a binder, a coating agent, an extender, a lubricant, a glidant, a flavor, or the like.

The pharmaceutical composition may further comprise, in addition to the above-described active ingredient, one or more pharmaceutically acceptable carrier for administration.

The pharmaceutical composition may be in the form of granule, powder, tablet, coated tablet, capsule, suppository, solution, syrup, juice, suspension, emulsion, drip, injectable solution, etc. For example, for preparation into tablet or capsule, the active ingredient may be combined with a nontoxic, pharmaceutically acceptable, inert carrier such as ethanol, glycerol, water, etc. Also, an adequate binder, lubricant, disintegrant or colorant may be included if desired or necessary. The binder may include starch, gelatin, natural sugar such as glucose or β-lactose, corn sweetener, natural or synthetic gum such as acacia, tragacanth or sodium oleate, sodium stearate, magnesium stearate, sodium benzoate, sodium acetate, sodium chloride, or the like, although not being limited thereto. The disintegrant may include starch, methyl cellulose, agar, bentonite, xanthan gum, or the like, although not being limited thereto.

When the composition is prepared into liquid solution, a pharmaceutically acceptable carrier suitable for sterilization can be selected from saline, sterile water, Ringer's solution, buffered saline, albumin injection solution, dextrose solution, maltodextrin solution, glycerol, ethanol, or a mixture thereof. If necessary, the composition may include other typical additives such as antioxidant, buffer or bacteriostat. Further, a diluent, a dispersant, a surfactant, a binder or a lubricant may be additionally added to prepare the composition into injection such as aqueous solution, suspension or emulsion, pill, capsule, granule or tablet.

Furthermore, the composition may be prepared into appropriate forms depending on particular disease or component according to the methods disclosed in Remington's Pharmaceutical Science (Mack Publishing Company, Easton, PA).

The pharmaceutical composition of the present disclosure may be administered orally or parenterally. When administered parenterally, it may be administered intravenously, subcutaneously, intramuscularly, intraabdominally or transdermally. Specifically, it may be administered orally.

An adequate dosage of the pharmaceutical composition of the present disclosure may be determined depending on various factors such as method of formulation, method of administration, a patient's age, body weight and gender, pathological condition, diet, administration time, administration route, excretion rate and response sensitivity. An ordinarily skilled physician may easily determine a dosage effective for the desired prevention or treatment. In an exemplary embodiment of the present disclosure, a daily dosage of the pharmaceutical composition of the present disclosure is 0.001-10 g/kg.

The pharmaceutical composition of the present disclosure may be prepared into unit dosage form or multiple dosage form using a pharmaceutically acceptable carrier and/or excipient according to methods commonly employed in the art. The formulation may be in the form of solution in oil or aqueous medium, suspension, emulsion, extract, powder, granule, tablet or capsule, and a dispersant or a stabilizer may be further included.

The present disclosure further provides a food composition for preventing or improving sleep disorder comprising rice bran or rice hull extract as an active ingredient.

The food composition according to the present disclosure may be prepared according to the same method as that of the pharmaceutical composition for use as functional food or food additive. The composition of the present disclosure may be added to, for example, beverages, alcoholic beverages, confectionery, diet bar, dairy products, meats, chocolate, pizza, instant noodles, other noodles, gums, ice creams, vitamin complexes, dietary supplements, or the like.

The food composition of the present disclosure may include, in addition to rice bran extract or rice bran powder as the active ingredient, ingredients commonly added when preparing foods. For example, proteins, carbohydrates, fats, nutrients, seasoning and flavor may be included. Examples of the carbohydrate may include common sugars including monosaccharides such as glucose, fructose, etc., disaccharides such as maltose, sucrose, oligosaccharide, etc. and polysaccharides such as dextrin, cyclodextrin, etc., and sugar alcohols such as xylitol, sorbitol, erythritol, etc. The flavor may be a natural flavor [thaumatin, stevia extract (e.g., rebaudioside A, glycyrrhizin, etc.]) or a synthetic flavor (saccharin, aspartame, etc.). For example, when the food composition of the present disclosure is in the form of drink or beverage, citric acid, high-fructose corn syrup, sugar, glucose, acetic acid, malic acid, fruit juice or various other plant extracts may be further included in addition to the rice bran or rice hull extract of the present disclosure.

The present disclosure provides a health functional food comprising the food composition for use in preventing or improving sleep disorder comprising the rice bran or rice hull extract as an active ingredient. The health functional food refers to a food prepared by adding rice bran extract or rice bran powder to foodstuffs such as beverages, teas, spices, gums, confectionery, etc. followed by encapsulation, trituration, suspension, or the like, providing a specific health benefit but without the side effect that may occur when a pharmaceutical drug is taken for a long period of time. The health functional food of the present disclosure is very useful since it can be taken safely. The addition amount of the rice bran extract or rice bran powder to the health functional food may be different depending on the particular health functional food. The amount may be within the range not affecting the original taste of the food. Usually, the rice bran extract or rice bran powder may be added in an amount of 0.01-50 wt%, specifically 0.1-20 wt%. When the health functional food is in the form of pill, granule, tablet or capsule, it may be added in an amount of usually 0.1-100 wt%, specifically 0.5-80 wt%. In an exemplary embodiment, the health functional food of the present disclosure may be in the form of pill, tablet, capsule or drink.

The present disclosure further provides a use of rice bran or rice hull extract for preparation of a drug or food for preventing, treating or improving sleep disorder. As described above, the rice bran extract or rice bran powder may be used to prevent, treat or improve sleep disorder.

The present disclosure further provides a method for preventing, treating or improving sleep disorder, comprising administering an effective amount of rice bran or rice hull extract to a mammal.

As used herein, the term "mammal" refers to a mammal which is the subject of treatment, observation or experimentation, specifically human.

As used herein, the term "effective amount" refers to an amount of the active ingredient or pharmaceutical composition that will elicit a biological or medical response in a tissue, system, animal or human that is being sought by a researcher, veterinarian, medical doctor or clinician, including, e.g., ameliorating the symptoms of the corresponding disease or disorder. Those skilled in the art will readily understand that the effective amount of the active ingredient and the number of administration will vary depending on the desired effect. Accordingly, the optimum administration dosage may be easily determined by those skilled in the art. It may be adjusted depending on various factors including the particular disease, severity of the disease, contents of the active ingredient and other ingredients included in the composition, formulation type, age, body weight, general physical conditions and gender of a patient, administration time, administration route, excretion rate, administration period and drug(s) used in combination. For an adult, the rice bran or rice hull extract may be administered with a daily dosage of 0.001 mg/kg-10 g/kg, once or several times a day.

The composition comprising the rice bran or rice hull extract as an active ingredient may be administered orally, rectally, intravenously, intraarterially, intraabdominally, intramuscularly, intrasternally, transdermally, locally, intraocularly or intradermally.

### EXAMPLES

### [Experimental methods]

### Test animals

ICR mice (18-22 g, male) and SD rats (200-250 g, male) were acquired from Koatech and Orient Bio, respectively, and accustomed for a week in cages before carrying out experiments. The animals were maintained under the condition of 23 ± 1 °C, humidity 55 ± 5%, 12/12-hr light/dark cycles (lighting from 9 am to 9 pm, 3000 lux), and feed and drinking water were given freely. All the animals were managed according to the guidelines of the Korea Food Research Institutional Animal Care and Use Committee (KFRI-IACUC).

### Pentobarbital-induced sleep test

Pentobarbital-induced sleep test was carried out at regular hours between 1 pm and 5 pm. Ten (n = 10) mice per each group were fasted for 24 hours before the test. All the test samples were prepared using 0.5% CMC-saline and administered orally (p.o.) to the mice 45 minutes before the administration of pentobarbital. The normal control group was treated with 0.5% CMC-saline at 10 mg/kg. Diazepam, one of the typical sleeping drugs, was used as positive control for comparison of the sleep-improving effect. Pentobarbital (Hanlim Pharm.) was administered intraabdominally (i.p.) at 45 mg/kg (hypnotic dosage) according to the experimental design. After the pentobarbital treatment, the mice were transferred to separated spaces and sleep latency and sleep duration were measured. The sleep latency was determined by the time until the righting reflex is lost for 1 minute or longer, and the sleep duration was determined by the time until the righting reflex is restored. The mouse which showed no sleeping behavior even 10 minutes after the administration of pentobarbital was excluded from the test.

### Analysis of sleep architecture

After an accommodation period of 1 week, electrodes were inserted into the Sprague-Dawley (SD) rats (200-250 g) for measurement of electroencephalograms (EEG) and electromyograms (EMG). After anesthetizing the rat with pentobarbital (50 mg/kg, i.p.), the head was fixed in a stereotaxic instrument. After incising the subcutaneous connective tissue, stainless steel screws and silver electrode lines were inserted for EEG and EMG recording. After fixing with dental cement followed by suturing and disinfection, antibiotics were injected for 3 days to prevent inflammation. The mice were allowed to recover for 7 days. For accommodation to the measurement environment, 0.5% CMC-saline (control) was orally administered (p.o.) and the recording apparatus was connected, from 4 days prior to the measurement. After orally administering the sample and waiting for 5 minutes for sedation, EEG and EMG were recorded for 6 hours, from 10:00 to 16:00, using PAL-8200 (Pinnacle Technology Inc., Oregon, USA). Sampling rate was set at 200 Hz (epoch time: 10 s). Filtering range was 0.1-25 Hz for EEG and 10-100 Hz for EMG. Sleep architecture was analyzed according to the fast Fourier transform (FFT) algorithm using SleepSign (Ver. 3.0, Kissei Comtec, Nagono, Japan). The result was represented by dividing into wake, rapid eye movement (REM) sleep (theta band: 6-10 Hz) and non-REM sleep (delta band: 0.65-4 Hz). Sleep latency was determined by the time until non-REM sleep with 10-sec epoch occurs consecutively at least 12 times.

### Reference Preparation Example 1: Preparation of rice extract

Rice water extract (RWE) was prepared by adding distilled water (1 L) to pulverized rice (100 g) and heating for 1 hour at 100 °C, and rice ethanol extract (REE) was prepared by adding 10 times (w/v) of 70% ethanol to pulverized rice and extracting for 1 day in a 50 °C incubator, followed by ultrasonication for 90 minutes 3 times. The resulting extracts were filtered, concentrated under reduced pressure, lyophilized and prepared into powder. The yield was 1.16 wt% for RWE and 0.19 wt% for REE.

### Preparation Example 2: Preparation of rice hull extract

Rice hull water extract (HWE) was prepared by adding distilled water (1 L) to pulverized rice hull (100 g) and heating for 1 hour at 100 °C, and rice hull ethanol extract (HEE) was prepared by adding 10 times (w/v) of 70% ethanol to pulverized rice hull and extracting for 1 day in a 50 °C incubator, followed by ultrasonication for 90 minutes 3 times. The resulting extracts were filtered, concentrated under reduced pressure, lyophilized and prepared into powder. The yield was 5% for HWE and 0.96% for HEE.

### Preparation Example 3: Preparation of rice bran extract

Rice bran water extract (BWE) was prepared by adding distilled water (1 L) to pulverized rice bran (100 g) and heating for 1 hour at 100 °C, and rice bran ethanol extract (BEE) was prepared by adding 10 times (w/v) of 70% ethanol to pulverized rice bran and extracting for 1 day in a 50 °C incubator, followed by ultrasonication for 90 minutes 3 times. The resulting extracts were filtered, concentrated under reduced pressure, lyophilized and prepared into powder. The yield was 5.56% for BWE and 7.02% for BEE.

Since BEE contained a lot of oily components, two fractions were further obtained in addition to BEE, which were named as BEE-Wax and BEE-Oil. That is to say, the rice bran extract was obtained as three fractions. Initially, the rice bran extract was separated into an upper layer and a lower layer. The upper layer is an oil fraction of the rice bran extract (BEE-Oil) and the lower layer is BEE. When the lower layer was further separated via centrifugation or through filter paper, it was separated into a liquid phase (liquid fraction of the rice bran extract) and a solid residue. The residue is a wax fraction of the rice bran extract (BEE-Wax).

### Reference Test Example 1: Pentobarbital-induced sleep test using rice extract

After orally administering (p.o.) the RWE and REE prepared in Preparation Example 1 at 50, 100, 250 and 500 mg/kg, pentobarbital was administered (45 mg/kg, i.p.) to induce sleep. As seen from FIG. 1 and FIG. 2, RWE resulted in decreased sleep latency and increased sleep duration in a concentration-dependent manner (p < 0.01), except for 50 mg/kg. Also, as seen from FIG. 3 and FIG. 4, REE resulted in decreased sleep latency and increased sleep duration in a concentration-dependent manner, except for 50 mg/kg. RWE resulted in more increase of sleep duration than REE, and decrease of sleep latency was similar for RWE and REE.

### Test Example 2: Pentobarbital-induced sleep test using rice hull extract

After orally administering (p.o.) the HWE and HEE prepared in Preparation Example 2 at 50, 100, 250 and 500 mg/kg, pentobarbital was administered (45 mg/kg, i.p.) to induce sleep. As seen from FIG. 5 and FIG. 6, HWE resulted in significantly decreased sleep latency and significantly increased sleep duration at 250 and 500 mg/kg in a concentration-dependent manner (p < 0.01). Also, as seen from FIG. 7 anrwed FIG. 8, HEE resulted in significantly decreased sleep latency (p < 0.01) and increased sleep duration in a concentration-dependent manner (p < 0.01), except for 50 mg/kg. HWE resulted in more increase of sleep duration than HEE, and decrease of sleep latency was similar for HWE and HEE.

### Test Example 3: Pentobarbital-induced sleep test using rice bran extract

After orally administering (p.o.) the BWE and BEE prepared in Preparation Example 3 at 50, 100, 250 and 500 mg/kg, pentobarbital was administered (45 mg/kg, i.p.) to induce sleep. BWE resulted in significantly decreased sleep latency (p < 0.01, FIG. 9), more than the positive control DZP. And, BWE resulted in significantly increased sleep duration in a concentration-dependent manner, except for 50 and 100 mg/kg (FIG. 10). Also, BEE resulted in decreased sleep latency, more than DZP (FIG. 11), and increased sleep duration in a concentration-dependent manner at 250 and 500 mg/kg (FIG. 12). Both BEE and BWE resulted in significantly decreased sleep latency and significantly increased sleep duration at 250 and 500 mg/kg, with no significant difference between BEE and BWE.

Also, in order to test the sleep-improving effect of BEE-Wax and BEE-Oil, after orally administering (p.o.) BEE-Wax at 50, 100, 250 and 500 mg/kg and BEE-Oil at 500 and 1,000 mg/kg, pentobarbital was administered intraabdominally (45 mg/kg, i.p.). When the change in sleep latency and sleep duration was measured, a surprising result was found for BEE-Wax. It resulted in decreased sleep latency (p < 0.01, FIG. 13) and increased sleep duration (p < 0.01, FIG. 14) in a concentration-dependent manner, even more than those of BEE. Also, BEE-Oil resulted in significantly decreased sleep latency (p < 0.01, FIG. 15) and increased sleep duration (p < 0.01, FIG. 16) at 1,000 mg/kg. Although the sleep-improving effect was not higher than that of BEE or BEE-Wax, BEE-Oil also had a sleep-improving effect.

### Test Example 4: Analysis of sleep architecture for rice bran extract

Sleep latency and total sleep duration after the oral administration of BEE 500 mg/kg are shown in FIG. 17. Mean sleep latency of the control was 31.9 minutes and that of BEE was 26.2 minutes, about 5.7 minutes shorter. Total sleep duration was 165.4 minutes for the control and 248.6 minutes for BEE 500 mg/kg, about 83 minutes longer.

Wake time, REM sleep time and non-REM sleep time of SD rat are shown in FIG. 18. The BEE-administered group exhibited increased non-REM sleep time and decreased wake time. BEE 500 mg/kg resulted in non-REM sleep time of 234.8 minutes, about 81 minutes increased from that of the control group, 153.8 minutes. The large part of the increased total sleep duration was non-REM sleep.

FIG. 19 shows change in wake, non-REM sleep and REM sleep with time in rat to which BEE 500 mg/kg was orally administered. Non-REM sleep increased greatly for 3 hours after the oral administration. It decreased slightly thereafter and then increased again. This suggests that BEE induced sleep as it was digested for the first 3 hours. Considering that the proportion of non-REM sleep was significantly higher than that of the control in all times, it can be seen that the sleep-improving effect lasted for 6 hours. Although BEE did not result in a significant decrease of sleep latency, it improved sleep by increasing total sleep duration.

### Preparation Example 4: Preparation of solvent fractions of BEE

BEE (liquid fraction of the BEE prepared in Preparation Example 3) was further separated using 4 solvents, hexane, butanol, water and ethyl acetate (FIG. 20).

BEE (200 kg) was extracted at 50 °C by immersing in 60% EtOH aqueous solution for 43 hours. The extract was filtered and the residue was further extracted 3 times by the same method. All the filtrates were combined and concentrated under reduced pressure to obtain EtOH extract (20 L). After adding 7 L of H₂O, the obtained EtOH extract was partition extracted using n-hexane (27 L × 2) / H₂O (27 L). The H₂O fraction was further partition extracted with EtOAc (27 L × 2), and the resulting H₂O fraction was further partition extracted with n-BuOH (25 L × 2). After concentration under reduced pressure, n-hexane fraction (BEE-H, 2176 g), EtOAc fraction (BEE-E, 1458 g), n-BuOH fraction (BEE-B, 1223 g) and H₂O fraction (BEE-W, 6.74 kg) were obtained (FIG. 20).

### Test Example 5: Pentobarbital-induced sleep test using solvent fractions of BEE

In order to test sleep-improving effect, after orally administering (p.o.) BEE-H, BEE-B, BEE-W and BEE-E at 50 and 250 mg/kg, pentobarbital was administered intraabdominally (45 mg/kg, i.p.) and the change in sleep latency and sleep duration was measured (FIG. 21 and FIG. 22).

BEE-E had no sleep-improving effect. Although it resulted in significant decrease of sleep latency, no significant increase of sleep duration was observed. BEE-W resulted in significantly decreased sleep latency (*p <* 0.01) and increased sleep duration (p < 0.01) at 250 mg/kg, but the effect was lower than that of BEE-H or BEE-B. BEE-H and BEE-B resulted in significantly decreased sleep latency (p < 0.01) and concentration-dependently increased sleep duration (p < 0.01). BEE-H showed better sleep-improving effect than BEE-B. Thus, BEE-H was further separated.

### Preparation Example 5: Preparation of sub-fractions of BEE-H

Among the solvent fractions of BEE, the n-hexane fraction (BEE-H), which showed significant activity in in-vivo test, was further separated into sub-fractions. BEE-H (365 g) was subjected to SiO₂ column chromatography (c.c.). A column with a diameter of 13 cm and a height of 15 cm was filled with silica gel resin. A mixture of n-hexane and EtOAc was gradually diluted from n-hexane:EtOAc = 10:1 to 7:1 and 2:1 for use as the eluent. As a result, a total of 12 sub-fractions (BEE-H-1 through BEE-H-12) were obtained (see FIG. 23).

### Test Example 6: Pentobarbital-induced sleep test using sub-fractions of BEE-H

In order to test sleep-improving effect, after orally administering (p.o.) BEE-H-1 through BEE-H-11 at 50 and 250 mg/kg, pentobarbital was administered intraabdominally (45 mg/kg, i.p.) and the change in sleep latency and sleep duration was measured.

As seen from FIG. 24 and FIG. 25, at 50 mg/kg, only BEE-H-2 resulted in significant increase of sleep duration (p < 0.01). And, only BEE-H-2, BEE-H-6, BEE-H-10 and BEE-H-11 resulted in significant decrease of sleep latency (p < 0.05). At 250 mg/kg, as seen from FIG. 26 and FIG. 27, BEE-H-1, BEE-H-2, BEE-H-4, BEE-H-7, BEE-H-10 and BEE-H-11 resulted in significant increase of sleep duration (p < 0.01), and all the sub-fractions excluding BEE-H-9 and BEE-H-10 resulted in significant decrease of sleep latency (*p* < 0.05, *p* < 0.01). To conclude, BEE-H-2, BEE-H-10 and BEE-H-11 showed superior sleep-improving effect among the 11 sub-fractions.

### Preparation Example 6: Preparation of sub-fractions of BEE-H sub-fractions

### (1) BEE-H-2 sub-fractions

As described above, BEE-H-2, BEE-H-10 and BEE-H-11 showed high activity in in-vivo test among the 12 sub-fractions of BEE-H (BEE-H-1 through BEE-H-12). Secondary sub-fractions were prepared from BEE-H-2 among them. BEE-H-2 (8.2 g) was subjected to ODS c.c. (ϕ 4 × 7 cm, acetone:H₂O = 1:1). The resulting fractions were combined and concentrated. 5 sub-fractions (BEE-H-2-1 through BEE-H-2-5) were obtained and identified by SiO₂ and ODS TLC (FIG. 28).

### (2) BEE-H-10 sub-fractions

Secondary sub-fractions were prepared from BEE-H-10. BEE-H-10 (9.5 g) was subjected to ODS c.c. (ϕ 6 × 17 cm, n-hexane:EtOAc = 10:1, 6:1, 4:1, 2:1 and 1:1, CHCl₃:MeOH = 8:1 and 1:1). 23 sub-fractions (BEE-H-10-1 through BEE-H-10-23) were obtained and identified by SiO₂ and ODS TLC (FIG. 29).

### (3) BEE-H-11 sub-fractions

Secondary sub-fractions were prepared from BEE-H-11. BEE-H-11 (810 mg) was subjected to ODS c.c. (ϕ 5 × 10 cm, n-hexane:EtOAc = 10:1, 5:1, 3:1 and 1:1, CHCl₃-MeOH = 8:1, 5:1 and 1:1). 13 sub-fractions (BEE-H-11-1 through BEE-H-11-13) were obtained and identified by SiO₂ and ODS TLC (FIG. 30).

### Test Example 7: Inhibition of activity of G protein-coupled receptors (GPCRs) by BEE-H-2

Inhibition of GPCR activity by BEE-H-2 was analyzed by Millipore Corporation (USA) according to the GPCRProfiler™ method. As a result, BEE-H-2 inhibited the activity of 7 GPCR receptors (serotonin 1A receptor, adenosine 1 receptor, histamine 1 receptor, histamine 2 receptor, acetylcholine 2 receptor, vasopressin 1A receptor and neuropeptide Y2 receptor) by at least 50% (FIG. 31).

### Test Example 8: Sleep-improving mechanism of rice or rice bran extract

In order to identify the sleep-improving mechanism of RWE prepared in Preparation Example 1 and BEE prepared in Preparation Example 3, the effect of the histamine receptor agonist 2-pyridylethylamine dihydrochloride (PD) on the sleep-improving of RWE and BEE was investigated.

The sleep-improving effect of a histamine receptor antagonist is inhibited by the histamine receptor agonist. In order to identify the sleep-improving mechanism, pyrilamine maleate salt (PMS) was used as the histamine receptor antagonist and PD was used as the agonist.

Each of RWE and BEE (500 mg/kg) and PMS (70 mg/kg) were orally administered and pentobarbital (hypnotic dosage, 45 mg/kg) was administered 45 minutes later. PD (20 mg/kg) was intraabdominally injected 10 minutes before the administration of RWE or BEE and PMS. Then, sleep latency and sleep duration were measured.

As seen from FIGS. 32-35, the sleep-improving effect of PMS was completely inhibited by PD. Also, the effect of RWE and BEE was inhibited by the histamine receptor agonist PD.

In order to investigate the effect of the agonist PD on the sleep-improving effect of diazepam (DZP), which is a GABAA-benzodiazepine agonist and one of the representative sleeping drugs, DZP (0.5 mg/kg), BEE (500 mg/kg) and PMS (70 mg/kg) were orally administered and pentobarbital (hypnotic dosage, 45 mg/kg) was administered 45 minutes later. The histamine receptor agonist PD (20 mg/kg) or the GABAA-benzodiazepine antagonist flumazenil (FLU, 8 mg/kg) was intraabdominally injected 10 minutes before the administration of DZP, BEE and PMS. Then, sleep latency and sleep duration were measured.

As seen from FIG. 36 and FIG. 37, PD did not affect the sleep latency or sleep duration of DZP. And, as seen from FIG. 38 and FIG. 39, FLU had an effect on the sleep-improving effect of DZP but not on the sleep-improving effect of BEE.

Thus, it can be seen that BEE improves sleep not by binding to the GABAA-benzodiazepine receptor but by inhibiting the histamine receptor.

Since such a natural product acting on the histamine receptor is not common, the rice bran or rice hull extract of the present disclosure may be used not only as an antihistamine to prevent or treat sleep disorder, anxiety and depression but also as a drug or food effective for preventing and treating the diseases mediated by the histamine receptor, such as allergic rhinitis, inflammatory bowel disease, asthma, bronchitis, nausea, gastric and duodenal ulcer, gastroesophageal reflux disease, or the like.

The rice bran or rice hull extract according to the present disclosure provides comparable or better effect of decreasing sleep latency, increasing sleep duration and increasing non-REM sleep as compared to diazepam, which is currently used as sleeping drug. It acts as a natural antihistamine since the sleep-improving effect of the rice bran or rice hull extract is inhibited by the histamine receptor agonist PD just as that of PMS is completely inhibited by PD. Derived from the natural product rice bran or rice hull, it has no side effect such as cognitive impairment, resistance or dependency even after long-term use.

## Claims

1. A pharmaceutical composition for use in the prevention or treatment of sleep disorder, comprising rice bran extract or rice hull extract as an active ingredient.

2. The pharmaceutical composition for use according to claim 1, wherein the rice bran or rice hull extract is an extract of rice bran or rice hull extracted in water, an organic solvent or a mixture thereof.

3. The pharmaceutical composition for use according to claim 2, wherein the organic solvent is one or more solvent selected from a group consisting of lower alcohol, hexane, acetone, ethyl acetate, chloroform and diethyl ether, and the organic solvent is preferably ethanol.

4. The pharmaceutical composition for use according to claim 1, wherein the rice bran or rice hull extract is one or more selected from a group consisting of a liquid fraction of rice bran or rice hull extract and a wax fraction of rice bran or rice hull extract.

5. The pharmaceutical composition for use according to claim 1, wherein the rice bran or rice hull extract is a fraction obtained by refractionating an organic solvent extract of rice bran or rice hull with a second organic solvent,
wherein the extract is preferably a fraction obtained by refractionating a lower alcohol extract of rice bran with a second organic solvent, and
wherein the rice bran or rice hull extract is more preferably a fraction obtained by refractionating an ethanol extract of rice bran with hexane.

6. The pharmaceutical composition according to claim 1 for use in the prevention or treatment of sleep disorder by reducing sleep latency, increasing sleep duration or increasing non-REM sleep.

7. A food composition for use in preventing or improving sleep disorder, which comprises rice bran or rice hull extract as an active ingredient.

8. The food composition for use according to claim 7, wherein the prevention or improvement of sleep disorder is to reduce sleep latency, increase sleep duration or increase non-REM sleep.

9. The food composition for use according to claim 7, wherein the rice bran or rice hull extract is an extract of rice bran or rice hull extracted in water, an organic solvent or a mixture thereof.

10. The food composition for use according to claim 8, wherein the organic solvent is one or more solvent selected from a group consisting of lower alcohol, hexane, acetone, ethyl acetate, chloroform and diethyl ether; and wherein the organic solvent is preferably ethanol.

11. The food composition for use according to claim 7, wherein the rice bran or rice hull extract is one or more selected from a group consisting of a liquid fraction of rice bran or rice hull extract and a wax fraction of rice bran or rice hull extract.

12. The food composition for use according to claim 7, wherein the rice bran or rice hull extract is a fraction obtained by refractionating an ethanol extract of rice bran or rice hull with hexane.

## Patentansprüche

1. Pharmazeutische Zusammensetzung zur Verwendung in der Prävention oder Behandlung von Schlafstörung, umfassend einen Reiskleie- oder Reisspreuestrakt als Wirkstoff.

2. Pharmazeutische Zusammensetzung zur Verwendung nach Anspruch 1, wobei der Reiskleie- oder Reisspreuextrakt ein Extrakt von Reiskleie oder Reisspreu, extrahiert in Wasser, einem organischen Lösungsmittel oder einer Mischung davon, ist.

3. Pharmazeutische Zusammensetzung zur Verwendung nach Anspruch 2, wobei das organische Lösungsmittel ein oder mehrere Lösungsmittel ist, ausgewählt aus einer Gruppe, bestehend aus niederem Alkohol, Hexan, Aceton, Ethylacetat, Chloroform und Diethylether, und das organische Lösungsmittel bevorzugt Ethanol ist.

4. Pharmazeutische Zusammensetzung zur Verwendung nach Anspruch 1, wobei der Reiskleie- oder Reisspreuextrakt ein oder mehrere ist, ausgewählt aus einer Gruppe, bestehend aus einer Flüssigfraktion von Reiskleie- oder Reisspreuextrakt und einer Wachsfraktion von Reiskleie- oder Reisspreuextrakt.

5. Pharmazeutische Zusammensetzung zur Verwendung nach Anspruch 1, wobei der Reiskleie- oder Reisspreuextrakt eine Fraktion ist, erhalten durch Refraktionieren eines organischen Lösungsmittelextrakts von Reiskleie oder Reisspreu mit einem zweiten organischen Lösungsmittel,
wobei der Extrakt bevorzugt eine Fraktion ist, gewonnen durch Refraktionieren eines niederen Alkoholextrakts von Reiskleie mit einem zweiten organischen Lösungsmittel, und
wobei der Reiskleie- oder Reisspreuextrakt bevorzugter eine Fraktion ist, erhalten durch Refraktionieren eines Ethanolextrakts von Reiskleie mit Hexan.

6. Pharmazeutische Zusammensetzung nach Anspruch 1 zur Verwendung in der Prävention oder Behandlung von Schlafstörung durch Verminderung von Schlaflatenz, Erhöhen von Schlafdauer oder Erhöhen von Nicht-REM-Schlaf.

7. Nahrungszusammensetzung zur Verwendung in der Prävention oder Verbesserung von Schlafstörung, die Reiskleie- oder Reisspreuextrakt als einen Wirkstoff umfasst.

8. Nahrungszusammensetzung zur Verwendung nach Anspruch 7, wobei die Prävention oder Verbesserung von Schlafstörung ist, Schlaflatenz zu vermindern, Schlafdauer zu erhöhen oder Nicht-REM-Schlaf zu erhöhen.

9. Nahrungszusammensetzung zur Verwendung nach Anspruch 7, wobei der Reiskleie- oder Reisspreuextrakt ein Extrakt von Reiskleie oder Reisspreu, extrahiert in Wasser, einem organischen Lösungsmittel oder einer Mischung davon, ist.

10. Nahrungszusammensetzung zur Verwendung nach Anspruch 8, wobei das organische Lösungsmittel ein oder mehrere Lösungsmittel ist, ausgewählt aus einer Gruppe, bestehend aus niederem Alkohol, Hexan, Aceton, Ethylacetat, Chloroform und Diethylether, und wobei das organische Lösungsmittel bevorzugt Ethanol ist.

11. Nahrungszusammensetzung zur Verwendung nach Anspruch 7, wobei der Reiskleie- oder Reisspreuextrakt um ein oder mehrere ist, ausgewählt aus einer Gruppe, bestehend aus einer Flüssigfraktion von Reiskleie- oder Reisspreuextrakt und einer Wachsfraktion von Reiskleie- oder Reisspreuextrakt.

12. Nahrungszusammensetzung zur Verwendung nach Anspruch 7, wobei der Reiskleie- oder Reisspreuextrakt eine Fraktion ist, erhalten durch Refraktionieren eines Ethanolextrakts von Reiskleie oder Reisspreu mit Hexan.

## Revendications

1. Composition pharmaceutique destinée à être utilisée dans la prévention ou le traitement des troubles du sommeil, comprenant un extrait de son de riz ou un extrait de balle de riz à titre de principe actif.

2. Composition pharmaceutique pour son utilisation selon la revendication 1, dans laquelle l'extrait de son de riz ou de balle de riz est un extrait de son de riz ou de balle de riz extrait dans l'eau, dans un solvant organique ou un mélange de ceux-ci.

3. Composition pharmaceutique pour son utilisation selon la revendication 2, dans laquelle le solvant organique est un ou plusieurs solvants choisis dans le groupe constitué par un alcool inférieur, l'hexane, l'acétone, l'acétate d'éthyle, le chloroforme et l'éther diéthylique, et le solvant organique est de préférence l'éthanol.

4. Composition pharmaceutique pour son utilisation selon la revendication 1, dans laquelle l'extrait de son de riz ou de balle de riz est un ou plusieurs extraits choisis dans le groupe constitué par la fraction liquide de l'extrait de son de riz ou de balle de riz et la fraction cireuse de l'extrait de son de riz ou de balle de riz.

5. Composition pharmaceutique pour son utilisation selon la revendication 1, dans laquelle l'extrait de son de riz ou de balle de riz est une fraction obtenue par refractionnement d'un extrait à un solvant organique du son de riz ou de la balle de riz à l'aide d'un second solvant organique,
dans laquelle l'extrait est de préférence une fraction obtenue par refractionnement d'un extrait à un alcool inférieur du son de riz à l'aide d'un second solvant organique, et
dans laquelle l'extrait de son de riz ou de balle de riz est plus préférablement une fraction obtenue par refractionnement d'un extrait à l'éthanol du son de riz ou de la balle de riz à l'aide d'un hexane.

6. Composition pharmaceutique selon la revendication 1 pour son utilisation dans la prévention ou le traitement des troubles du sommeil par réduction du délai d'endormissement, accroissement de la durée du sommeil ou accroissement du sommeil lent.

7. Composition alimentaire destinée à être utilisée dans la prévention ou l'amélioration des troubles du sommeil, qui comprend un extrait de son de riz ou de balle de riz à titre de principe actif.

8. Composition alimentaire pour son utilisation selon la revendication 7, dans laquelle la prévention ou l'amélioration des troubles du sommeil consiste à réduire le délai d'endormissement, à accroître la durée du sommeil ou à accroître le sommeil lent.

9. Composition alimentaire pour son utilisation selon la revendication 7, dans laquelle l'extrait de son de riz ou de balle de riz est un extrait de son de riz ou de balle de riz extrait dans l'eau, dans un solvant organique ou un mélange de ceux-ci.

10. Composition alimentaire pour son utilisation selon la revendication 8, dans laquelle le solvant organique est un ou plusieurs solvants choisis dans le groupe constitué par un alcool inférieur, l'hexane, l'acétone, l'acétate d'éthyle, le chloroforme et l'éther diéthylique ; et dans laquelle le solvant organique est de préférence l'éthanol.

11. Composition alimentaire pour son utilisation selon la revendication 7, dans laquelle l'extrait de son de riz ou de balle de riz est un ou plusieurs extraits choisis dans le groupe constitué par la fraction liquide de l'extrait de son de riz ou de balle de riz et la fraction cireuse de l'extrait de son de riz ou de balle de riz.

12. Composition alimentaire pour son utilisation selon la revendication 7, dans laquelle l'extrait de son de riz ou de balle de riz est une fraction obtenue par refractionnement d'un extrait à l'éthanol du son de riz ou de la balle de riz à l'aide d'un hexane.
